(19) **Europäisches Patentamt / European Patent Office / Office européen des brevets**

(11) **EP 1 599 837 B1**

(12) # EUROPEAN PATENT SPECIFICATION

(45) Date of publication and mention of the grant of the patent:
**21.02.2007 Bulletin 2007/08**

(21) Application number: **04709292.9**

(22) Date of filing: **09.02.2004**

(51) Int Cl.:
*G06T 11/00* (2006.01)

(86) International application number:
**PCT/IB2004/000365**

(87) International publication number:
**WO 2004/072904 (26.08.2004 Gazette 2004/35)**

(54) **SYSTEM AND METHOD FOR EXACT IMAGE RECONSTRUCTION FOR HELICAL CONE BEAM COMPUTED TOMOGRAPHY INCLUDING REDUNDANT DATA**

SYSTEM UND VERFAHREN FÜR EXAKTE BILDREKONSTRUKTION FÜR SPIRAL-Kegelstrahl-COMPUTERTOMOGRAPHIE MIT REDUNDANten DATEN

SYSTEME ET PROCEDE POUR LA RECONSTRUCTION EXACTE D'IMAGES POUR LA TOMOGRAPHIE PAR ORDINATEUR DE FAISCEAU conique hélicoïdale COMPRENANT DES DONNEES REDONDANTES

(84) Designated Contracting States:
**AT BE BG CH CY CZ DE DK EE ES FI FR GB GR HU IE IT LI LU MC NL PT RO SE SI SK TR**

(30) Priority: **14.02.2003 US 447428 P**
**27.06.2003 US 483165 P**

(43) Date of publication of application:
**30.11.2005 Bulletin 2005/48**

(73) Proprietor: **Koninklijke Philips Electronics N.V.**
**5621 BA Eindhoven (NL)**

(72) Inventors:
• **HEUSCHER, Dominic, J.**
**Cleveland, Ohio 44143 (US)**
• **BROWN, Kevin, M.**
**Cleveland, Ohio 44143 (US)**

(74) Representative: **Bosma, Rudolphus Hubertus Antonius**
**Philips**
**Intellectual Property & Standards**
**P.O. Box 220**
**5600 AE Eindhoven (NL)**

(56) References cited:
• **NOO FREDERIC ET AL: "Exact helical reconstruction using native cone-beam geometries" PHYS. MED. BIOL.;PHYSICS IN MEDICINE AND BIOLOGY DEC 7 2003, vol. 48, no. 23, 7 December 2003 (2003-12-07), pages 3787-3818, XP002279259**
• **KATSEVICH A: "Analysis of an exact inversion algorithm for spiral cone-beam CT" PHYSICS IN MEDICINE AND BIOLOGY, TAYLOR AND FRANCIS LTD. LONDON, GB, vol. 47, no. 15, 30 October 2001 (2001-10-30), pages 2583-2597, XP002272932 ISSN: 0031-9155**
• **ALEXANDER KATSEVICH: "Microlocal analysis of an FBP algorithm for truncated spiral cone beam data" JOURNAL OF FOURIER ANALYSIS AND APPLICATIONS, vol. 8, no. 5, September 2002 (2002-09), pages 407-425, XP002279450 Birkhäuser, Boston**

**Description**

The following relates to the diagnostic imaging arts. It finds particular application in helical conebeam computed tomography imaging, and will be described with particular reference thereto. However, it also finds application in other types of tomographic imaging.

Exact conebeam reconstruction methods have been developed which fulfill all the requirements of the three-dimensional Radon transform. For example, an exact conebeam reconstruction method has been developed by Katsevich (see for example Katsevich et al, Proceedings SPIE Medical Imaging Conference, San Diego, California (February 2003)). The Katsevich technique removes any redundant data and does not incorporate it.

In inexact three-dimensional reconstruction, redundant data is often filtered and combined. This is what is done in two-dimensional reconstructions, as in, for example, U.S. Patent 4,293,912 to Walters, wherein data extending beyond opposite ends of a 180° plus fan single slice data set are weighted and combined. In the case of U.S. Patent 5,446,799 of Tuy, two-dimensional redundant data is combined to improve image resolution.

The document "Analysis of an exact inversion algorithm for spiral cone-beam CT", Alexander Katsevich, Physics in Medicine and Biology, Taylor and Francis LTD. London, GB, vol. 47, no. 15, 30 October 2001 (2001-10-30), pages 2583-2597, XP002272932 ISSN: 0031-9155 discloses an exact filtered back projection inversion formula for cone-beam spiral CT. This algorithm uses only projection data within an exact reconstruction window for the filtered back projection.

In the document "Microlocal analysis of FBP algorithm for truncated spiral cone beam data", Alexander Kasevich, Journal of Fourier Analysis and Applications, vol. 8, no. 5, September 2002 (2002-09), pages 407-425, XP002279450 Birkhäuser, Boston a further filtered back projection algorithm for inversion of spiral cone-beam data of a CT system (computed tomography system) is disclosed.

The present invention contemplates an improved apparatus and method that overcomes the aforementioned limitations and others.

The invention is defined by the independent claims. The dependent claims define advantageous embodiments.

Incorporation of redundant data into the reconstruction is advantageous for at least two reasons. First, use of redundant data in helical reconstructions provides a continuous transition of projection data in both time and angle, significantly reducing artifacts due to data inconsistency (for example, due to anatomic motion) between ends of the reconstructed data set. Second, for a generally rectangular detector aperture, a substantial portion of the acquired projection data falls outside the pi-window or other exact reconstruction window, adversely impacting dose utilization. The substantial benefits of incorporating redundant data into the reconstruction have been demonstrated by comparison of inexact 3-pi versus pi reconstructions. Similar benefits of redundant data incorporation can be expected for exact reconstructions.

One advantage resides in improved transitions in time and angle across exact reconstruction windows.

Another advantage resides in improved dose utilization though incorporation of redundant data into exact conebeam reconstruction.

Numerous additional advantages and benefits will become apparent to those of ordinary skill in the art upon reading the following detailed description of the preferred embodiments.

The invention may take form in various components and arrangements of components, and in various process operations and arrangements of process operations. The drawings are only for the purpose of illustrating preferred embodiments and are not to be construed as limiting the invention.

FIGURE 1 diagrammatically shows a helical conebeam computed tomography imaging system including an exact reconstruction processor that incorporates redundant data.

FIGURE 2 shows an exemplary source-focused curved detector geometry.

FIGURE 3 diagrammatically shows components of the hybrid convolution processor of FIGURE 1.

FIGURE 4 diagrammatically shows several preferred redundant data sets for incorporation into the reconstruction.

FIGURE 5 compares exemplary K-planes and complementary K-planes.

FIGURE 6 shows selection of suitable aperture weighting functions G(w) for including 100%, 33%, and 0% of redundant data into the reconstruction.

FIGURE 7 shows selection of suitable aperture weighting functions G(w) for voxels at different positions relative to the helical axis.

FIGURE 8 diagrammatically shows an image reconstruction process that incorporates redundant data as a null data set.

FIGURE 9 shows aperture functions suitable for performing the image reconstruction process of FIGURE 5 using the exact hybrid reconstruction processor of FIGURE 1.

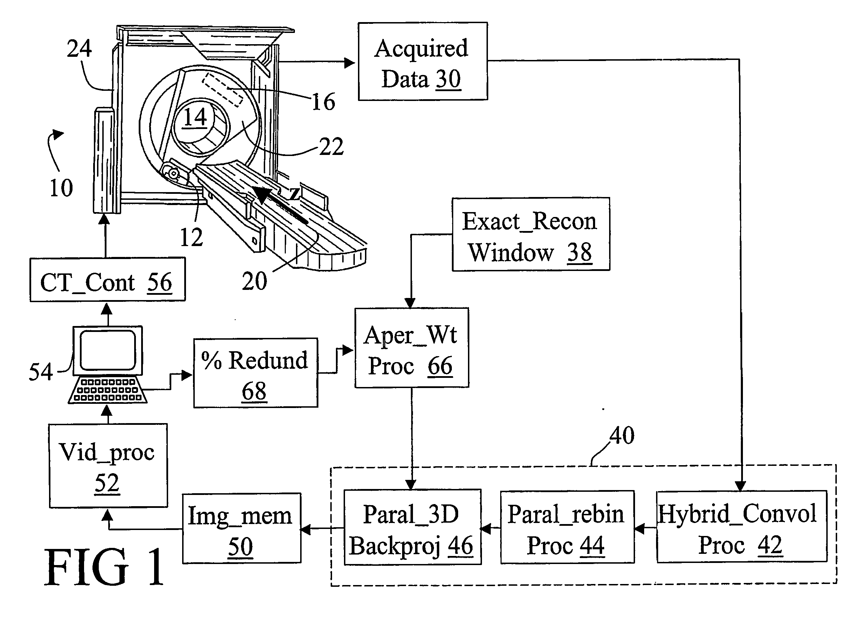

With reference to FIGURE 1, a helical conebeam computed tomography imaging scanner **10** includes an x-ray source **12** that projects an x-ray conebeam into an examination region **14**. After passing through the examination region, the x-ray conebeam is detected by a two-dimensional x-ray detector **16** (shown diagrammatically in phantom in FIGURE 1) that includes an array of detector elements arranged to detect the x-ray conebeam after passing through the examination region **14.**

To effect a helical trajectory of the x-ray source **12** about an imaging subject, the imaging subject is placed on a couch **20** or other support. The couch moves linearly along a z- or longitudinal direction as indicated. The x-ray source **12** and the x-ray detector **16** are oppositely mounted respective to the examination region **14** on a rotating gantry **22,** such that rotation of the gantry **22** effects rotation of the x-ray source **12,** and hence rotation of the conebeam. Rotation of the gantry **22** along with simultaneous, continuous linear motion of the couch **20** effects a helical trajectory of the x-ray source **12** and the x-ray conebeam around the imaging subject disposed on the couch **20.**

The x-ray detector **16** is shown mounted on the rotating gantry **22** such that it rotates along with the x-ray source **12** to intercept the x-ray conebeam throughout the helical trajectory. However, it is also contemplated to replace the x-ray detector **16** by an x-ray detector band mounted around a stationary gantry **24.**

In operation, during helical orbiting of the x-ray source **12** relative to the imaging subject, the x-ray conebeam is projected into the examination region **14** where it interacts with the imaging subject. Some portion of the x-rays are absorbed by the imaging subject to produce a generally spatially varying attenuation of the x-ray conebeam. The x-ray detector **16** measures the x-ray intensities across the conebeam to generate x-ray absorption data that is stored in an acquired projection data memory **30.**

Projection data within an exact reconstruction window **38** is optionally exactly reconstructed by an exact reconstruction processor **40** that implements an exact reconstruction that fulfills the requirements of the three-dimensional Radon transform. In a preferred embodiment, the exact reconstruction processor **40** includes a hybrid convolution processor **42,** a parallel rebinning processor **44,** and a parallel three-dimensional backprojector **46** that cooperate to perform exact reconstruction in native scan coordinates. However, another exact conebeam reconstruction can be employed, such as the method of Katsevich (see for example Katsevich et al, Proceedings SPIE Medical Imaging Conference, San Diego, California (February 2003)) which employs a voxel-based coordinate system.

The exactly reconstructed image is stored in an image memory **50** and is suitably processed by a video processor **52** to generate a three-dimensional rendering, one or more image slices, or other visual representation of the reconstructed image that is displayed on a video display of a user interface **54.** Rather than a video display, the image representation can be formatted by a printer driver and printed out using a printer, transmitted over an electronic network, stored electronically, or otherwise processed. Preferably, the user interface **54** communicates with a computed tomography controller **56** to enable a radiologist or other operator to initiate imaging or otherwise control operation of the computed tomography scanner **10.**

Although the exact reconstruction processor **40** can exactly reconstruct projection data within the exact reconstruction window **38** without incorporating redundant data, the resulting image representation may be degraded due to motion artifacts or noise. To reduce these effects, the reconstruction preferably incorporates redundant projection data residing outside the exact reconstruction window **38** around peripheries of aperture edges of the exact reconstruction window **38.**

Preferably, the backprojector **46** is an aperture-weighted backprojector that applies aperture weighting to projection data during the backprojecting. An aperture weighting processor **66** assigns weighting values to the projection data based on a position of the projections respective to the exact reconstruction window **38.** Preferably, the aperture weighting processor 66 assigns aperture weighting values selected to be substantially zero beyond a transition region at the peripheries the exact reconstruction window **38** and substantially unity inside the exact reconstruction window **38** and outside the transition region, with the transition region being a smooth and symmetric aperture weighting transition region therebetween. The size of the transition region of the aperture weighting function is selected based on a desired percentage **68** of redundant data to be incorporated into the reconstruction. The radiologist or other operator can select, via the user interface **54,** to use 0% redundant data, that is, reconstruct only data within the exact reconstruction window **38,** or the radiologist or other operator can select some or up to 100% of the redundant data collected by the physical detector **16.**

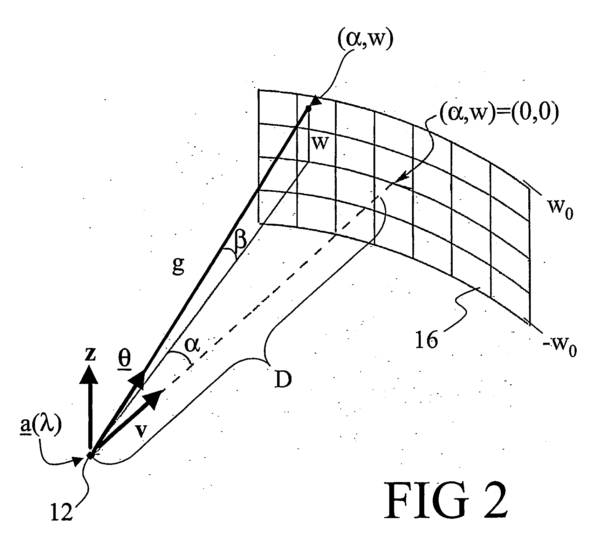

A preferred embodiment of the exact backprojection processor **40** operates in native scan coordinates. FIGURE 2 diagrams native scan coordinates for a preferred source-focused curved detector geometry that comports with radiation detectors commonly used in conebeam computed tomography. A projection fan coordinate $\alpha$ indicates the projection angle in the fan angle direction, while a projection cone angle coordinate $\beta$ indicates the projection angle in the cone angle direction. With the x-ray source **12** at a position $\underline{a}(\lambda)$ where $\lambda$ is a helix angle of the x-ray source **12,** a projection g lies along a projection direction vector $\theta$ and has coordinates $g(\lambda, \alpha, w)$ where w is a coordinate in the cone angle direction given by $w = D \tan(\beta)$ where D is a source-to-detector distance from the x-ray source **12** to a center of the detector **16.** The coordinate w is parallel to the axial or z-direction. The lower and upper edges of the detector 16 are designated by $-w_0$ and $w_0$, respectively. The center of the curved detector **16** corresponds to projection $g(\lambda, 0, 0)$, that is, $\alpha = w = 0$, and has a direction vector $\underline{\theta} = \underline{v}$. The detector **16** is not curved along the cone angle direction, but is curved along the fan direction, that is, along the direction corresponding to the fan coordinate $\alpha$. The detector curvature along

the angle coordinate $\alpha$ is selected so that all detector elements for a given angle coordinate $\beta$ are substantially equidistant from the x-ray source **12**. That is, the detector curvature along the angle coordinate $\alpha$ is source-focused.

The preferred exact backprojection processor **40** is described with exemplary reference to the source-focused curved detector geometry diagrammed in FIGURE 2. However, those skilled in the art can adapt the conebeam reconstruction processor **40** to a flat detector geometry or other detector geometry. Moreover, the backprojection processor **40** employing a hybrid convolution processor **42** operating in native scan coordinates can be replaced by another exact reconstruction processor, such as one that implements the exact reconstruction of Katsevich which operates in a voxel-based coordinate system. Still further, the redundant data incorporation methods described herein can be practiced with other substantially exact reconstruction processors. For example, the Wedge reconstruction algorithm of Tuy 5,446,799 has not been shown to fulfill all the requirements of the three-dimensional Radon transform; however, images reconstructed by the Wedge algorithm without redundant data can be visually indistinguishable from images reconstructed using reconstructions known to be exact.

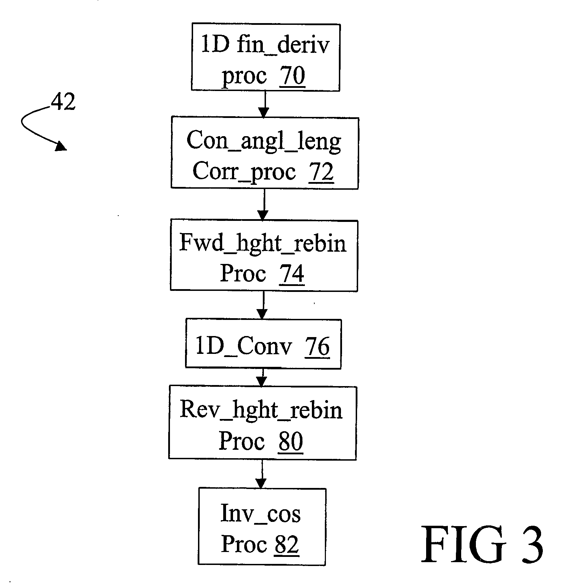

With returning reference to FIGURE 1 and with further reference to FIGURE 3, the hybrid convolution processor **42** performs a hybrid convolution including a differentiation convolution along the projection direction $\underline{\theta}$ and a one-dimensional convolution with respect to $\alpha$ in a forward height-rebinned geometry.

A one-dimensional finite derivative processor **70** performs a one-dimensional derivative along the helix angle $\lambda$ at constant projection direction $\underline{\theta}$ according to:

$$g_{h1}(\lambda,\alpha,w) = g'(\lambda,\underline{\theta}(\lambda,\alpha,w)) = \lim_{\varepsilon \to 0}\frac{g(\lambda+\varepsilon,\underline{\theta}) - g(\lambda,\underline{\theta})}{\varepsilon} \qquad (1).$$

The derivative expressed in Equation (1) is preferably implemented as a convolution using a discrete finite difference approach, although other numerical differentiation methods known to the art can be employed. A cone angle length correction processor **72** normalizes projection lengths according to:

$$g_{h2}(\lambda,\alpha,w) = \cos(\beta)\, g_{h1}(\lambda,\alpha,w) = \frac{D}{\sqrt{D^2 + w^2}}\, g_{h1}(\lambda,\alpha,w) \qquad (2).$$

The differentiated and length-normalized projection data is rebinned with respect to K-planes $K(\lambda,\psi)$ by a forward height rebinning processor **74** to get constant $\psi$ surfaces according to:

$$g_{h3}(\lambda,\alpha,\psi) = g_{h2}(\lambda,\alpha,w_\kappa(\alpha,\psi)) \qquad (3),$$

where

$$w_\kappa(\alpha,\psi) = \frac{DP}{2\pi R}\left(\psi\cos(\alpha) + \frac{\psi}{\tan(\psi)}\sin(\alpha)\right) \qquad (4).$$

Equations (3) and (4) are applied over all $\psi$ in a range $[-\pi/2-\alpha_m,\ \pi/2+\alpha_m]$ where $\alpha_m$ is a fan angle defined by the size $R_{fov}$ of the field of view and the helix radius R, that is, $\alpha_m = \arcsin(R_{fov}/R)$. The height-rebinned data is convolved by an FFT convolution processor **76** that performs a one-dimensional convolution with respect to $\alpha$ at a fixed angle $\psi$ according to:

$$g_{h4}(\lambda,\alpha,\psi) = h_h(\sin(\alpha)) \otimes g_{h3}(\lambda,\alpha,\psi) \qquad (5)$$

where $\otimes$ is a convolution operator and $h_h(s) = 1/s$ is a Hilbert convolution kernel. A reverse height rebinning processor **80** rebins the convolved projection data according to:

$$g_{h5}(\lambda,\alpha,w) = g_{h4}(\lambda,\alpha,\psi_\kappa(\alpha,w)) \tag{6},$$

where $\psi_\kappa$ is the angle $\psi$ of smallest absolute value that satisfies the equation:

$$w = \frac{DP}{2\pi R}\left(\psi\cos(\alpha) + \frac{\psi}{\tan(\psi)}\sin(\alpha)\right) \tag{7}.$$

The rebinning processor **80** is optionally replaced by another rebinning processor that provides a suitable rebinning for facilitating incorporation of a selected amount of redundant data using a one-dimensional aperture weighting function. An inverse cosine weighting processor **82** weights the projection data according to:

$$g_{h6}(\lambda,\alpha,w) = g_5(\lambda,\alpha,w)/\cos(\alpha) \tag{8}.$$

The parallel rebinning processor **44** rebins the convolved projection data $g_{h6}(\lambda,\alpha,w)$ into a parallel geometry according to:

$$g^F(\lambda_w,u,w) = g_6(\lambda_w + a\sin(u/R), a\sin(u/R), w) \tag{9},$$

and the filtered and rebinned projection data $g^F(\lambda_w,u,w)$ are backprojected by the aperture-weighted backprojector **46** according to:

$$f(\underline{x}) = BP_{\lambda_i,\lambda_o}\left[\frac{g^F(\lambda_w, u*(\lambda_w,\underline{x}), w*(\lambda_w,\underline{x})) \cdot G(w*(\lambda_w,\underline{x}))}{\sum_{\lambda_w' = \lambda_w + n\pi} G(w*(\lambda_w',\underline{x}))}\right] \tag{10},$$

where $\lambda_w$, $\lambda'_w \in (\lambda_i, \lambda_o)$ which corresponds to the maximum illuminated range for the voxel at $\underline{x}$ and $(u^*, w^*)$ are the interpolated projection coordinates for the projection $\lambda_w$ and voxel at x. The aperture weightings $G()$ computed by the aperture weighting processor **66**. Preferred aperture weightings will be described below.

The described reconstruction processor **40** has been shown by comparison with the exact voxel-based reconstruction of Katsevich to be an exact reconstruction which fulfills all the requirements of the three-dimensional Radon transform for a pi-window. Advantageously, the described reconstruction operates in native scan coordinates and incorporates the aperture-weighted parallel three-dimensional backprojector **46**. A suitable aperture-weighted three-dimensional parallel backprojector **46** is described in U.S. patent application serial no. 10/274,816 by Heuscher et al., filed on October 21, 2002.

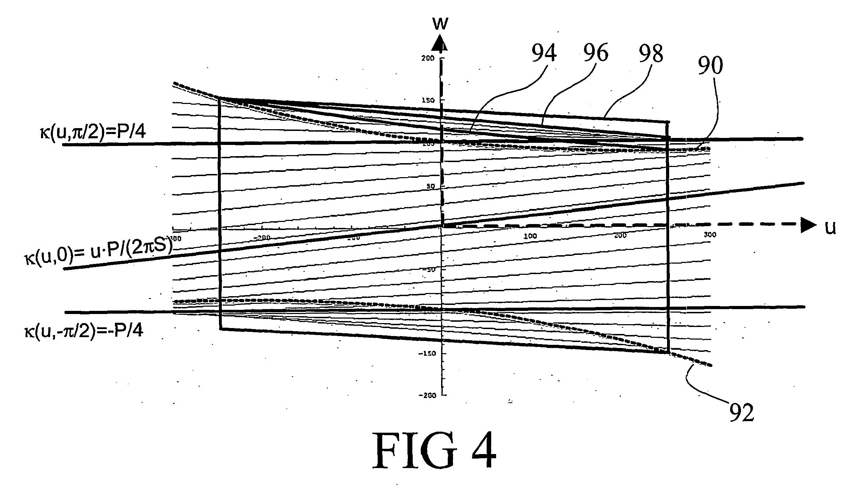

With reference to FIGURE 4, several preferred redundant projection data sets are described. The coordinate u in FIGURE 4 corresponds to the fan coordinate $\alpha$ after the parallel rebinning performed by the parallel rebinning processor **44,** while the coordinate w is the aperture coordinate in the cone angle direction described previously with reference to FIGURE 2. FIGURE 4 shows exemplary K-planes on the detector and also indicates top and bottom curved aperture edges **90, 92** of a pi-window that is suitable for use as the exact reconstruction window **38**. Each preferred redundant data set includes two symmetric parts: one above the top aperture edge **90,** and the other below the bottom aperture edge **92**. For convenience, only the portion of each redundant data set above the top pi-window aperture edge **90** is indicated in FIGURE 4, but the described redundant data selections will be appreciated as applying to the data sets below the lower pi-window aperture edge **92**.

A first preferred redundant projection data set is bounded by the aperture edge **90** and a straight line **94** in the parallel-rebinned geometry. The straight line **94** connects endpoints of the aperture edge **90** of the pi-window. This redundant projection data set is relatively small, and advantageously does not require additional rebinning operations beyond those performed by the parallel rebinning processor **44.**

A second preferred redundant projection data set has bound **96** corresponding to a last projection defmed by the K-planes. When using either of the redundant data sets having bounds **94, 96,** the forward height rebinning processor **74** of the hybrid convolution processor **42** preferably rebins the redundant data set to K-planes in the usual way, that is, according to Equations (3) and (4).

A third preferred redundant projection data set has bound **98** corresponding to a last projection defined by modified complementary K-planes. When using the third redundant data set having bound **98,** which includes more redundant data than the first and second preferred sets bounded by bounds **94, 96,** the forward height rebinning processor **74** of the hybrid convolution processor **42** preferably rebins the redundant data set to modified K-planes by replacing $g_{h3}(\lambda,\alpha,\psi)$ in the convolution of Equation (5) by $g_3(\lambda,\alpha,\psi')$ where the complementary modified K-planes designated by $\psi'$ are defined as:

$$w_{\kappa'}(\alpha,\psi'+\pi) = w_{\kappa'}(\alpha,\psi') + \cos(\alpha)P/2 \quad for \quad |\psi'| \leq \pi/2 \tag{11}.$$

and

$$w_{\kappa'}(\alpha,\psi'-\pi) = w_{\kappa'}(-\alpha,\psi') - \cos(\alpha)P/2 \quad for \quad |\psi'| \leq \pi/2 \tag{12}.$$

for the redundant data. Any projections that are truncated by the finite aperture of the detector 16 are preferably extrapolated according to:

$$g_3(\lambda,\alpha,\psi') = g_3(\lambda,\alpha,w_0) \quad for\ all\ (\alpha,\psi') \quad s.t. \left\{ \left| w_{\kappa'}(\alpha,\psi') \right| > w_0 \right\} \tag{13}.$$

where $w_0$ corresponds to the aperture edges of the x-ray detector **16** as shown in FIGURE 2.

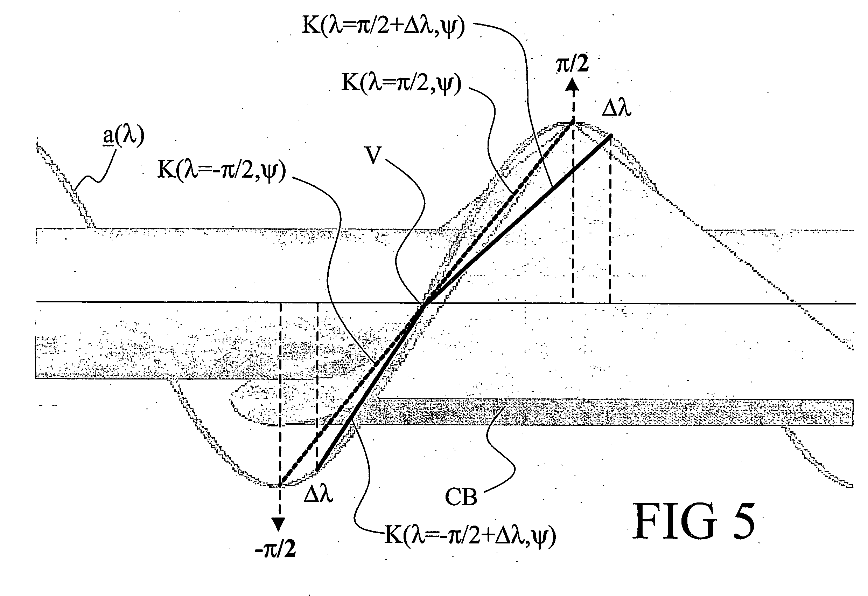

FIGURE 5 illustrates complementary K-planes defined by Equations (11)-(13). A voxel on the helical axis is measured using conebeam CB which follows helical trajectory $\underline{a}(\lambda)$. The K-planes $K(\lambda=-\pi/2,\psi)$ and $K(\lambda=\pi/2,\psi)$, which are shown on-edge in FIGURE 5 and represented by dotted lines in FIGURE 5, are fully complementary and coplanar. In contrast, the complementary K-planes $K(\lambda=-\pi/2+\Delta\lambda,\psi')$ and $K(\lambda=\pi/2+\Delta\lambda,\psi')$ indicated by the solid lines are not coplanar, leading to some inconsistency for $\Delta\lambda>0$. In other words, the complementary K-planes are bent or folded about an intersection line V containing the measured voxel. The complementary modified K-planes of Equations (11)-(13) can also be employed with the smaller first and second redundant data sets having bounds **94, 96.** Those skilled in the art can select other redundant data sets; however, the described preferred redundant data sets advantageously do not require additional rebinning operations.

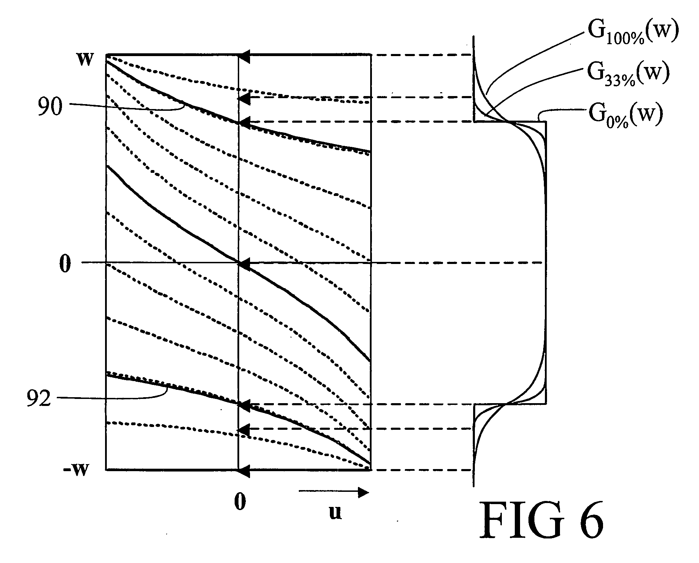

In employing any of the preferred redundant data sets described with reference to FIGURE 4, the aperture weighting processor **66** (see FIGURE 1) preferably applies a smooth normalized aperture weighting function G(w) that smooths transitions in the vicinity of aperture edges **90, 92.** After processing by the hybrid convolution processor **42,** the parallel rebinning processor **44** rebins the data (rebinned data shown as dotted curves in the aperture map of FIGURE 6) to a wedge or other suitable rebinned geometry so that a one-dimensional normalized aperture weighting can be used to combine the redundant data with data within the exact reconstruction window. The reverse height rebinning processor **80** can be adapted to provide suitable rebinning for the selected percentage of redundant data (which may be, for example, the amount of data corresponding to a selected bound **94, 96, 98** of FIGURE 4) to facilitate weighted normalization of the redundant data and complementary data using a one-dimensional aperture function G(w). A preferred form of the function G(w) satisfies the constraints of: G(w)=0 for $|w|=w_0$; G(w)=0.5 for $|w|=P/4$ (that is, at the edges **90, 92** of the exact reconstruction window); and G(w)=1 for $|w|<0.5P-w_0$. To account for data redundancy of complementary data separated by a 180° helical turn, the aperture weighting includes smoothly varying transition regions about the aperture positions $|w|=P/4$ to provide suitably weighted normalized combination of the redundant data.

FIGURE 6 shows exemplary weighting functions $G_{100\%}(w)$, $G_{33\%}(w)$, and $G_{0\%}(w)$ which are appropriate for inclusion of 100% of redundant data, 33% of redundant data, and 0% of redundant data, respectively, for a voxel on the helix axis. For 0% redundant data, $G_{0\%}(w)$ is a rectangular aperture weighting that retains data within the exact reconstruction window while discarding the redundant data outside the exact reconstruction window. In contrast, aperture weighting $G_{33\%}(w)$ includes a limited transition region that effects weighted combination of some redundant data from outside the exact reconstruction window. The reduced aperture weight in the transition region outside the exact reconstruction window versus complementary projection data residing in the transition region inside the exact reconstruction provides greater weight to the data inside the exact reconstruction window, but still allows some contribution from the redundant data outside the reconstruction window but near the aperture edges **90, 92.** Aperture weighting $G_{100\%}(w)$ provides for incorporation of more redundant data versus $G_{33\%}(w)$ by further increasing the width of the transition regions.

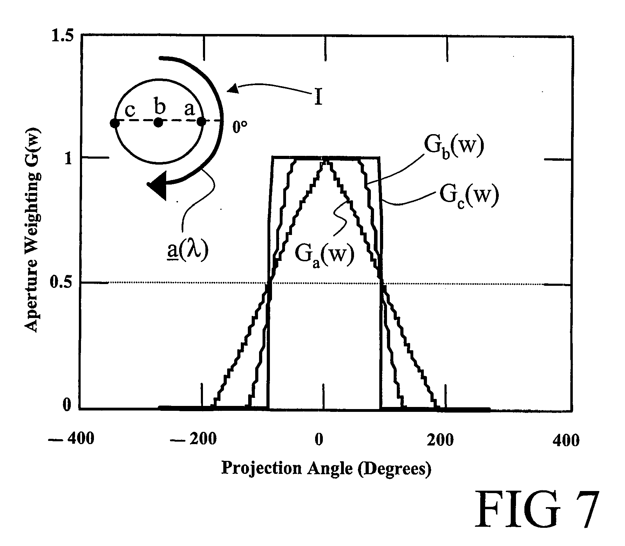

FIGURE 7 shows how the aperture weighting varies depending upon the position of the voxel relative to the helical axis. Inset **I** of FIGURE 7 diagrammatically shows the field of view (represented by a circle), a portion of the helical trajectory $\underline{a}(\lambda)$ employed in data acquisition for the voxels of interest, and three voxels labeled **a, b, c** positioned closest to the helical trajectory portion, on the helical axis, and furthest from the helical trajectory portion, respectively. The corresponding aperture weighting functions $G_a(w)$, $G_b(w)$, $G_c(w)$ are shown in the graph of FIGURE 7.

For the voxel **c,** limited redundant data is acquired due to the distance of voxel c from the x-ray source during data acquisition. The corresponding $G_c(w)$ has small transition regions and is close to corresponding to the exact reconstruction window. For the voxel **a** which is close to the helical trajectory portion, substantial redundant data is acquired and so $G_a(w)$ has very broad transition regions to incorporate the substantial redundant data. For voxel b which is intermediate between voxel **a** and voxel **c,** an intermediate aperture function $G_b(w)$ is appropriate. It will be observed that all the aperture weighting functions $G_a(w)$, $G_b(w)$, $G_c(w)$ are normalized such that G(w)=0.5 at the edges **90, 92** of the exact reconstruction window, G(w) rises smoothly toward unity near the center of the exact reconstruction window, and decreases smoothly toward zero outside the exact reconstruction window.

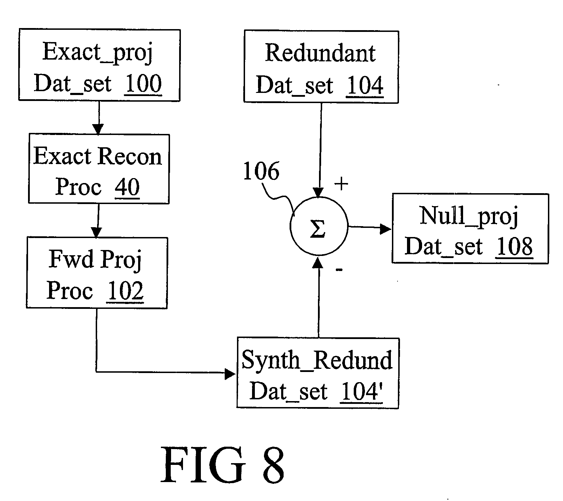

With reference to FIGURE 8, another suitable approach for incorporating redundant data into a reconstruction employing the exact reconstruction processor **40** is described. The approach of FIGURE 8 takes advantage of the capability of the exact reconstruction processor **40** (shown as a single block in FIGURE 8) to perform an exact reconstruction of an exact projection data set 100 lying within the exact reconstruction window **38.** The exactly reconstructed image is reprojected by a forward projection operator **102** over at least a range corresponding to an acquired redundant projection data set **104.** This reprojection produces a synthetic redundant data set **104'** over a range corresponding to the acquired redundant projection data set **104.**

Because the exact reconstruction processor **40** performs exact reconstruction of the exact projection data set, it follows that the synthetic projection data is identical to the exact projection data set **100** within the exact reconstruction window **38.** Moreover, in the absence of noise, motion artifacts, or other inconsistencies, the synthetic redundant projection data set **104'** in the range of the redundant data set **104** is identical to the redundant projection data set **104.** Thus, a combining block **106** suitably subtractively combines the synthetic redundant projection data set **104'** with the acquired redundant projection data set **104** to produce a null projection data set **108.** Combining the exact projection data set **100** and the null projection data set **108** in the exact reconstruction **40** thus provides improved continuity of projection data across time and angle transitions, which in turn reduces artifacts due to data inconsistency and can reduce noise by averaging over the additional redundant data embodied by the null data set **108.**

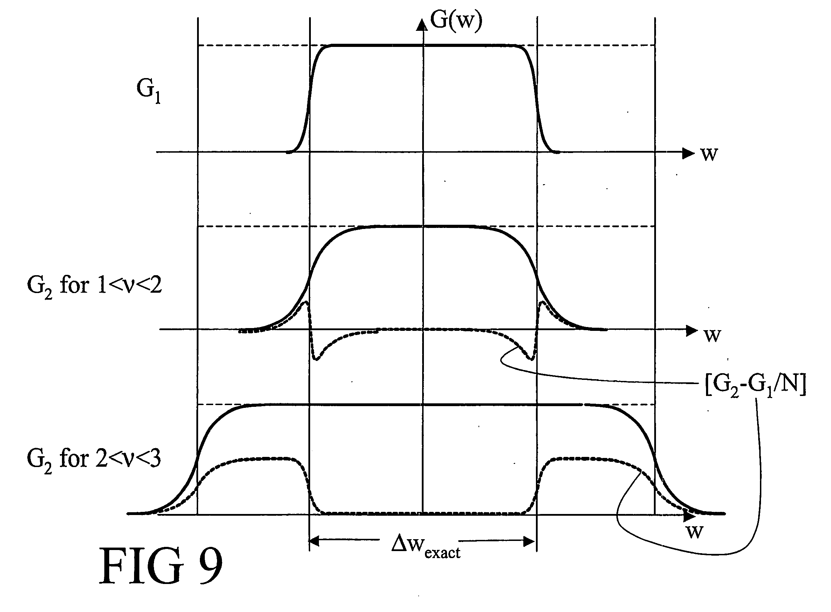

With returning reference to FIGURE 1 and further reference to FIGURE 9, a preferred approach for performing a v-pi reconstruction with a null projection data set is as follows. The hybrid convolution processor **42** convolves the acquired projection data and the rebinning processor **44** rebins the projection data to produce an exact hybrid convolved data set $P_1$ corresponding to the projections $g^F(\lambda_w,u,w)$ of Equation (9). The aperture weighting processor 66 and backprojector **46** perform aperture-weighted backprojection using a smooth weighting function $G_1(w)$ shown in FIGURE 9 having a passband $\Delta w_{exact}$ substantially corresponding to the aperture width of the exact reconstruction window **38.** The exact reconstructed image is re-projected by the forward projection processor **102** to define a synthetic projection data set $P_2$ that spans at least the exact reconstruction window **38** and the range of the redundant projection data set. A projection data set $P_1'$ is constructed by filtering the data set $P_1$ to make sure projections have the same spatial response as the synthetic projection data set $P_2$. The projection data set $(P_1'-P_2)$ is the null projection data set **108.** To account for the original transition region of the aperture weighting function $G_1(w)$, the final reconstructed image is preferably generated according to:

$$I_{final} = AWBP(G_1, P_1) + [AWBP(G_2, (P_1'-P_2)) - AWBP(G_1, (P_1'-P_2)) / N] \qquad (14),$$

where: $G_2(w)$ is a second aperture weighting function having an extended passband that is larger than the passband $\Delta w_{exact}$ and encompasses the exact reconstruction window 38 and the range of the redundant projection data set; AWBP() represents the aperture-weighted backprojection performed AWBP() according to Equation (10) by the backprojector 46; N is the largest integer less than v; and $I_{final}$ is the final reconstructed image with contributions from the exact projection data set and from the null projection data set.

FIGURE 9 shows two exemplary $G_2(w)$ weighting functions: one for a range 1<v<2; and one for a range 2<v<3. The term in brackets in Equation (14) is an image correction corresponding to the null data set weighted by $[G_2-G_1/N]$ after aperture-weighted normalization. The $[G_2-G_1/N]$ weighting is indicated in FIGURE 9 by dotted lines.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1.  A conebeam computed tomography imaging system including:

    a conebeam computed tomography scanning means (10) for acquiring oversampled conebeam projection data along a generally helical source trajectory around an examination region (14); and
    exact reconstruction means (40) including:

    a convolving means (42) for performing at least one convolution of the acquired projection data, the convolving operating on projection data falling within an exact reconstruction window (38) and on at least some redundant projection data falling outside the exact reconstruction window (38) to produce convolved projection data, and
    an aperture-weighted backprojecting means (46, 66) for performing aperture-weighted backprojecting of the convolved projection data using an aperture weighting function that weightedly combines at least some convolved redundant projection data with convolved projection data falling within the exact reconstruction window (38) to generate a reconstructed image with contributions from redundant projection data,

    wherein the aperture-weighted backprojecting means (46, 66) employs a smoothly varying aperture weighting function whose value is selected to be substantially zero outside the exact reconstruction window (38) and substantially unity inside the exact reconstruction window (38) with a smooth aperture weighting transition region therebetween.

2.  The imaging system as set forth in claim 1, wherein the convolving means (42) includes:

    a height rebinning means (74) for height-rebinning projections along K-planes.

3.  The imaging system as set forth in claim 2, wherein the height rebinning means (74) rebins redundant projection data falling outside the exact reconstruction window (38) to modified K-planes that are folded about a pi-line respective to K-planes corresponding to complementary projection data falling within the exact reconstruction window (3 8).

4.  The imaging system as set forth in claim 2, wherein the height rebinning means (74) rebins redundant projection data falling outside the exact reconstruction window (38) to K-planes that are complementary to K-planes corresponding to projection data falling within the exact reconstruction window (38).

5.  The imaging system as set forth in claim 2, wherein the exact reconstruction window (38) is a pi-window, and the aperture-weighted backprojecting means (46, 66) employs an aperture weighting function that combines redundant projection data lying between an edge (90, 92) of the pi-window and a line (94) connecting endpoints of the edge of the pi-window with complementary projection data lying within the pi-window.

6.  The imaging system as set forth in claim 1, wherein the aperture-weighted backprojecting means (46, 66) employs a smoothly varying aperture weighting function G(w) that satisfies the criteria:

$$G(w) = 0 \qquad \text{at} \qquad |w| = w_0,$$

$$G(w) = 0.5 \quad \text{at} \quad |w| = P/4,$$

and

$$G(w) = 1 \quad \text{at} \quad |w| \le (0.5P - w_0),$$

where w is a coordinate in the cone angle direction, wo corresponds to aperture edges of a radiation detector (16) of the conebeam computed tomography scanning means (10), and P corresponds to a helical pitch of the generally helical trajectory.

7. The imaging system as set forth in claim 1, further including:

a means (102) for forward projecting an exact reconstructed image formed by applying the exact reconstruction means (40) to an exact projection data set (100) falling within the exact reconstruction window (38) to generate simulated projection data corresponding to the convolved redundant projection data that is to be weightedly combined with convolved projection data falling within the exact reconstruction window (38); and
a means (106) for combining the simulated projection data and convolved projection data to generate a null projection data set (108).

8. The imaging system as set forth in claim 7, wherein:

in the applying of the exact reconstruction means (40) to the exact projection data set (100), the aperture weighted backprojecting means (46, 66) applies a first aperture weighting function ($G_1$) to the exact projection data set (100) to produce a non redundant exact reconstructed image, the first aperture weighting function ($G_1$) having a first aperture passband ($\Delta w_{exact}$) substantially corresponding to the exact reconstruction window (38).

9. The imaging system as set forth in claim 8, wherein:

the reconstructed image with contributions from redundant projection data is generated by the aperture-weighted backprojecting means (46, 66) by:

backprojecting the null projection data set (108) weighted by the first aperture weighting function ($G_1$) to generate a first null reconstructed image,
backprojecting the null projection data set (108) weighted by an extended aperture weighting function ($G_2$) having an extended aperture passband substantially encompassing both the exact reconstruction window (38) and at least some redundant data set to generate a second null reconstructed image, and
combining the nonredundant exact reconstructed image, the first null reconstructed image, and the second null reconstructed image.

10. The imaging system as set forth in claim 7, wherein the exact reconstruction means (40) reconstructs the null projection data set (108) into at least one null reconstructed image, the reconstructed image with contributions from redundant projection data being generated by combining an image reconstructed by the reconstructing means (40) from the convolved projection data and the at least one null reconstructed image.

11. The imaging system as set forth in claim 1, wherein the exact reconstruction means (40) performs a reconstruction that satisfies the requirements of the three-dimensional Radon transform for projection data falling within the exact reconstruction window (38).

12. A conebeam computed tomography imaging method including;

acquiring oversampled conebeam projection data along a generally helical source trajectory around an examination region (14); and
reconstructing acquired projection data falling within an exact reconstruction window (38) and at least some acquired redundant projection data falling outside the exact reconstruction window (38) into a reconstructed image with contributions from redundant projection data, the reconstructing including:

convolving the acquired projection data, the convolving operating on acquired projection data falling within the exact reconstruction window (38) and on at least some acquired redundant projection data falling outside the exact reconstruction window (38) to produce convolved projection data, and
performing aperture-weighted backprojecting of the convolved projection data using an aperture weighting function that weightedly combines at least some convolved redundant projection data with convolved projection data falling within the exact reconstruction window (38) to generate the reconstructed image with contributions from redundant projection data,

wherein the aperture-weighting function includes:

substantially zero weighting values outside the exact reconstruction window (38);
substantially unity weighting values inside the exact reconstruction window (38); and
weighting values smoothly varying between zero and unity in a transition region disposed at a periphery of the exact reconstruction window (38).

13. The imaging method as set forth in claim 12, wherein the convolving includes:

rebinning projection data to K-planes; and
performing at least two convolution operations, at least one of which is performed after the rebinning to K-planes.

14. The imaging method as set forth in claim 13, wherein the rebinning of projection data along K-planes includes:

rebinning projection data falling within the exact reconstruction window (38) to first K-planes; and
rebinning redundant projection data falling outside the exact reconstruction window (38) to modified K-planes that are complementary to at least some of the first K-planes.

15. The imaging method as set forth in claim 12, wherein the exact reconstruction window (38) is a pi-window, and the performing of aperture-weighted backprojecting includes:

weightedly combining convolved projection data lying outside the pi-window along a periphery of edges (90, 92) of the pi-window with convolved projection data falling within the exact reconstruction window (38).

16. The imaging method as set forth in claim 12, wherein the aperture-weighting function is a one-dimensional aperture weighting function.

17. The imaging method as set forth in claim 12, wherein the performing aperture-weighted backprojecting includes:

aperture-weighting the rebinned projection data using a normalized one-dimensional smooth aperture weighting function that normalizes contributions of complementary projections,

the aperture weighting function having:

smoothly varying normalization weighting values for projections in a transition region substantially centered on edges (90, 92) of the exact reconstruction window (38);
a substantially unity weighting value inside the exact reconstruction window (38) and outside the transition region; and
a substantially zero weighting value outside the exact reconstruction window (38) and outside the transition region.

18. The imaging method as set forth in claim 12, further including:

reconstructing an exact portion of projection data falling within the exact reconstruction window (38) into a nonredundant exact reconstructed image using a reconstruction that satisfies the requirements of the three-dimensional Radon transform;
forward projecting the nonredundant exact reconstructed image to generate a simulated projection data set extending outside the exact reconstruction window (38); and

combining the simulated projection data set and convolved projection data including at least some convolved redundant projection data to form a null projection data set (108).

19. The imaging method as set forth in claim 18, wherein the performing of aperture-weighted backprojecting of the convolved projection data using an aperture weighting function that weightedly combines at least some convolved redundant projection data with convolved projection data falling within the exact reconstruction window (38) includes:

reconstructing the null projection data set (108) into at least one null reconstructed image; and

combining the nonredundant exact reconstructed image and the at least one null reconstructed image (108).

20. The imaging method as set forth in claim 12, wherein the performing of aperture-weighted backprojecting of the convolved projection data using an aperture weighting function that weightedly combines at least some convolved redundant projection data with convolved projection data falling within the exact reconstruction window (38) includes :

reconstructing the acquired projection data weighted by a first aperture function substantially corresponding to the exact reconstruction window (38) into a nonredundant exact reconstructed image;

forward projecting the nonredundant exact reconstructed image to generate a simulated projection data set extending outside of the exact reconstruction window (38); and combining the simulated projection data set and the acquired projection data to form a null projection data set (108).

21. The imaging method as set forth in claim 20, wherein the performing of aperture-weighted backprojecting of the convolved projection data using an aperture weighting function that weightedly combines at least some convolved redundant projection data with convolved projection data falling within the exact reconstruction window (38) further includes:

reconstructing the null projection data set (108) weighted by an extended aperture function that spans a larger aperture range than the first aperture function into a first null reconstructedimage;

reconstructing the null projection data set (108) weighted by the first aperture function into a second null reconstructed image; and

combining the nonredundant exact reconstructed image, the first null reconstructed image, and the second null reconstructed image to form the reconstructed image with contributions from redundant projection data.

22. The imaging method as set forth in claim 21, wherein the combining of the nonredundant exact reconstructed image, the first null reconstructed image, and the second null reconstructed image includes:

subtractively combining the second null reconstructed image from the first null reconstructed image to generate an image correction; and

correcting the nonredundant exact reconstructed image by the image correction.

**Patentansprüche**

1. Kegelstrahl-Computertomographie-Bildgebungssystem, das Folgendes umfasst:

ein Kegelstrahl-Computertomographie-Abtastmittel (10) zum Erfassen von überabgetasteten Kegelstrahl-Projektionsdaten entlang einer im Allgemeinen spiralförmigen Trajektorie einer Quelle um eine Untersuchungsregion (14) herum; und

exakte Rekonstruktionsmittel (40), die Folgendes umfassen:

ein Faltungsmittel (42) zum Durchführen von mindestens einer Faltung der erfassten Projektionsdaten, wobei sich das Falten auf Projektionsdaten bezieht, die innerhalb eines exakten Rekonstruktionsfensters (38) liegen, und auf zumindest einige redundante Projektionsdaten, die außerhalb des exakten Rekonstruktionsfensters (38) liegen, um gefaltete Projektionsdaten zu erzeugen, und

ein apertur-gewichtetes Rückprojektionsmittel (46, 66) zum Durchführen einer apertur-gewichteten Rückprojektion der gefalteten Projektionsdaten unter Verwendung einer Apertur-Gewichtungsfunktion, die zumindest einige gefaltete redundante Projektionsdaten mit gefalteten Projektionsdaten gewichtet kombiniert, die innerhalb des exakten Rekonstruktionsfensters (38) liegen, um ein rekonstruiertes Bild mit Beiträgen von redundanten Projektionsdaten zu erzeugen,

wobei das apertur-gewichtete Rückprojektionsmittel (46, 66) eine fließend variierende Apertur-Gewichtungsfunktion verwendet, deren Wert so gewählt wird, dass er außerhalb des exakten Rekonstruktionsfensters (38) im Wesentlichen Null beträgt und innerhalb des exakten Rekonstruktionsfensters (38) im Wesentlichen Eins, mit einem glatten Apertur-Gewichtungs-Übergangsbereich dazwischen.

2. Bildgebungssystem nach Anspruch 1, in dem das Faltungsmittel (42) Folgendes umfasst:

ein Höhen-Rebinning-Mittel (74) für Höhen-Rebinning-Projektionen entlang der K-Ebenen.

3. Bildgebungssystem nach Anspruch 2, in dem das Höhen-Rebinning-Mittel (74) die redundanten Projektionsdaten, die außerhalb des exakten Rekonstruktionsfensters (38) liegen, einem Rebinning auf modifizierte K-Ebenen unterzieht, die um eine Pi-Linie gefaltet sind, beziehungsweise auf K-Ebenen, die den komplementären Projektionsdaten entsprechen, welche innerhalb des exakten Rekonstruktionsfensters (38) liegen.

4. Bildgebungssystem nach Anspruch 2, in dem das Höhen-Rebinning-Mittel (74) die redundanten Projektionsdaten, die außerhalb des exakten Rekonstruktionsfensters (38) liegen, einem Rebinning auf K-Ebenen unterzieht, die komplementär zu K-Ebenen sind, die den Projektionsdaten entsprechen, welche innerhalb des exakten Rekonstruktionsfensters (38) liegen.

5. Bildgebungssystem nach Anspruch 2, in dem das exakte Rekonstruktionsfenster (38) ein Pi-Fenster ist, und das apertur-gewichtete Rückprojektionsmittel (46, 66) eine Apertur-Gewichtungsfunktion verwendet, die redundante Projektionsdaten, die zwischen einer Kante (90, 92) des Pi-Fensters und einer Linie (94), die die Endpunkte der Kante des Pi-Fensters verbindet, liegen, mit komplementären Projektionsdaten kombiniert, die innerhalb des Pi-Fensters liegen.

6. Bildgebungssystem nach Anspruch 1, in dem das apertur-gewichtete Rückprojektionsmittel (46, 66) eine fließend variierende Apertur-Gewichtungsfunktion G(w) verwendet, die die nachfolgenden Kriterien erfüllt:

$$G(w) = 0 \quad \text{bei} \quad |w| = w_0,$$

$$G(w) = 0{,}5 \quad \text{bei} \quad |w| = P/4,$$

und

$$G(w) = 1 \quad \text{bei} \quad |w| \leq (0{,}5P - w_0).$$

wobei w eine Koordinate in Kegelwinkelrichtung ist, wo den Aperturkanten eines Strahlungsdetektors (16) des Kegelstrahl-Computertomographie-Abtastmittels (10) entspricht, und P einer spiralenförmigen Steigung einer im Allgemeinen spiralenförmigen Trajektorie entspricht.

7. Bildgebungssystem nach Anspruch 1, das weiterhin Folgendes umfasst:

ein Mittel (102) zur Vorwärtsprojektion eines exakt rekonstruierten Bildes, das durch Anwenden des exakten Rekonstruktionsmittels (40) auf einen exakten Projektionsdatensatz (100) gebildet wird, der innerhalb des exakten Rekonstruktionsfensters (38) liegt, um simulierte Projektionsdaten zu erzeugen, die den gefalteten redundanten Projektionsdaten entsprechen, welche gewichtet mit den gefalteten Projektionsdaten innerhalb des exakten Rekonstruktionsfensters (38) zu kombinieren sind; und
ein Mittel (106) zum Kombinieren der simulierten Projektionsdaten und gefalteten Projektionsdaten, um einen Nullprojektionsdatensatz (108) zu erzeugen.

8. Bildgebungssystem nach Anspruch 7, in dem:

das apertur-gewichtete Rückprojektionsmittel (46, 66) beim Anwenden des exakten Rekonstruktionsmittels (40) auf den exakten Projektionsdatensatz (100) eine erste Apertur-Gewichtungsfunktion $(G_1)$ auf den exakten Projektionsdatensatz (100) anwendet, um ein nicht-redundantes exakt rekonstruiertes Bild zu erzeugen, wobei die erste Apertur-Gewichtungsfunktion $(G_1)$ einen ersten Apertur-Durchlassbereich $(\Delta w_{exakt})$ umfasst, der im Wesentlichen dem exakten Rekonstruktionsfenster (38) entspricht.

9. Bildgebungssystem nach Anspruch 8, in dem:

das rekonstruierte Bild mit Beiträgen von den redundanten Projektionsdaten durch das apertur-gewichtete Rückprojektionsmittel (46, 66) erzeugt wird, durch:

Rückprojizieren des durch die erste Apertur-Gewichtungsfunktion $(G_1)$ gewichteten Nullprojektionsdatensatzes (108), um ein erstes null-rekonstruiertes Bild zu erzeugen,
Rückprojizieren des Nulldatensatzes (108), der durch eine erweiterte Apertur-Gewichtungsfunktion $(G_2)$ gewichtet wurde, welche über einen erweiterten Apertur-Durchlassbereich verfügt, der im Wesentlichen sowohl das exakte Rekonstruktionsfenster (38) als auch mindestens einige redundante Datensätze umfasst, um ein zweites null-rekonstruiertes Bild zu erzeugen, und
Kombinieren des nicht-redundanten exakt rekonstruierten Bildes, des ersten null-rekonstruierten Bildes und des zweiten null-rekonstruierten Bildes.

10. Bildgebungssystem nach Anspruch 7, in dem das exakte Rekonstruktionsmittel (40) den Nullprojektionsdatensatz (108) zu mindestens einem null-rekonstruierten Bild rekonstruiert, wobei das rekonstruierte Bild mit Beiträgen von den redundanten Projektionsdaten erzeugt wird, indem ein vom Rekonstruktionsmittel (40) aus den gefalteten Projektionsdaten rekonstruierten Bild mit dem zumindest einen null-rekonstruierten Bild kombiniert wird.

11. Bildgebungssystem nach Anspruch 1, in dem das exakte Rekonstruktionsmittel (40) eine Rekonstruktion durchführt, die die Anforderungen der dreidimensionalen Radon-Transformation für Projektionsdaten erfüllt, die innerhalb des exakten Rekonstruktionsfensters (38) liegen.

12. Kegelstrahl-Computertomographie-Bildgebungsverfahren, das Folgendes umfasst:

Erfassen überabgetasteter Kegelstrahl-Projektionsdaten entlang einer im Allgemeinen spiralförmigen Trajektorie einer Quelle um eine Untersuchungsregion (14) herum; und
Rekonstruieren erfasster Projektionsdaten, die innerhalb eines exakten Rekonstruktionsfensters (38) liegen und mindestens einiger erfasster redundanter Projektionsdaten, die außerhalb des exakten Rekonstruktionsfensters (38) liegen, zu einem rekonstruierten Bild mit Beiträgen von den redundanten Projektionsdaten, wobei das Rekonstruieren Folgendes umfasst:

Falten der erfassten Projektionsdaten, wobei sich das Falten auf erfasste Projektionsdaten bezieht, die innerhalb des exakten Rekonstruktionsfensters (38) liegen, und auf zumindest einige erfasste redundante Projektionsdaten, die außerhalb des exakten Rekonstruktionsfensters (38) liegen, um gefaltete Projektionsdaten zu erzeugen, und
Durchführen einer apertur-gewichteten Rückprojektion der gefalteten Projektionsdaten unter Verwendung einer Apertur-Gewichtungsfunktion, die zumindest einige gefaltete redundante Projektionsdaten mit gefalteten Projektionsdaten gewichtet kombiniert, die innerhalb des exakten Rekonstruktionsfensters (38) liegen, um das rekonstruierte Bild mit Beiträgen von den redundanten Projektionsdaten zu erzeugen,

wobei die Apertur-Gewichtungsfunktion Folgendes umfasst:

Gewichtungswerte von im Wesentlichen Null außerhalb des exakten Rekonstruktionsfensters (38);
Gewichtungswerte von im Wesentlichen Eins innerhalb des exakten Rekonstruktionsfensters (38), und
Gewichtungswerte, die in einem Übergangsbereich, der sich am äußeren Rand des exakten Rekonstruktionsfensters (38) befindet, fließend zwischen Null und Eins variieren.

13. Bildgebungsverfahren nach Anspruch 12, wobei das Falten Folgendes umfasst:

Rebinning der Projektionsdaten auf K-Ebenen; und

Durchführen von mindestens zwei Faltungsvorgängen, von denen zumindest einer nach dem Rebinning auf K-Ebenen durchgeführt wird.

14. Bildgebungsverfahren nach Anspruch 13, wobei das Rebinning der Projektionsdaten entlang der K-Ebenen Folgendes umfasst:

Rebinning der Projektionsdaten, die innerhalb des exakten Rekonstruktionsfensters (38) liegen, auf erste K-Ebenen; und

Rebinning der redundanten Projektionsdaten, die außerhalb des exakten Rekonstruktionsfensters (38) liegen, auf modifizierte K-Ebenen, die komplementär zu zumindest einigen der ersten K-Ebenen sind.

15. Bildgebungsverfahren nach Anspruch 12, wobei das exakte Rekonstruktionsfenster (38) ein Pi-Fenster ist, und das Durchführen der apertur-gewichteten Rückprojektion Folgendes umfasst:

gewichtetes Kombinieren gefalteter Projektionsdaten, die außerhalb des Pi-Fensters entlang eines äußeren Randes der Kanten (90, 92) des Pi-Fensters liegen, mit gefalteten Projektionsdaten, die innerhalb des exakten Rekonstruktionsfensters (38) liegen.

16. Bildgebungsverfahren nach Anspruch 12, wobei die Apertur-Gewichtungsfunktion eine eindimensionale Apertur-Gewichtungsfunktion ist.

17. Bildgebungsverfahren nach Anspruch 12, wobei das Durchführen der apertur-gewichteten Rückprojektion Folgendes umfasst:

Aperturgewichtung der dem Rebinning unterzogenen Projektionsdaten unter Verwendung einer normalisierten eindimensionalen glatten Apertur-Gewichtungsfunktion, die Beiträge der komplementären Projektionen normalisiert,

wobei die Apertur-Gewichtungsfunktion Folgendes beinhaltet:

fließendes Variieren der Normalisierungsgewichtungswerte für Projektionen in einem Übergangsbereich, der im Wesentlichen auf die Kanten (90, 92) des exakten Rekonstruktionsfensters (38) zentriert ist;

einen Gewichtungswert von im Wesentlichen Eins innerhalb des exakten Rekonstruktionsfensters (38) und außerhalb des Übergangsbereichs; und

einen Gewichtungswert von im Wesentlichen Null außerhalb des exakten Rekonstruktionsfensters (38) und außerhalb des Übergangsbereichs.

18. Bildgebungsverfahren nach Anspruch 12, das weiterhin Folgendes umfasst:

Rekonstruieren eines exakten Teils der Projektionsdaten, die innerhalb des exakten Rekonstruktionsfensters (38) liegen, zu einem nicht-redundanten exakt rekonstruierten Bild, unter Verwendung einer Rekonstruktion, die die Anforderungen der dreidimensionalen Radon-Transformation erfüllt;

Vorwärtsprojizieren des nicht-redundanten exakt rekonstruierten Bildes, um einen simulierten Projektionsdatensatz zu erzeugen, der sich außerhalb des exakten Rekonstruktionsfensters (38) erstreckt; und

Kombinieren des simulierten Projektionsdatensatzes mit den gefalteten Projektionsdaten, die zumindest einige gefaltete redundante Projektionsdaten beinhalten, um einen Nullprojektionsdatensatz (108) zu bilden.

19. Bildgebungsverfahren nach Anspruch 18, wobei das Durchführen der apertur-gewichteten Rückprojektion der gefalteten Projektionsdaten unter Verwendung einer Apertur-Gewichtungsfunktion, die zumindest einige gefaltete redundante Projektionsdaten mit gefalteten Projektionsdaten, die innerhalb des exakten Rekonstruktionsfensters (38) liegen, gewichtet kombiniert, Folgendes umfasst:

Rekonstruieren des Nullprojektionsdatensatzes (108) zu mindestens einem null-rekonstruierten Bild; und

Kombinieren des nicht-redundanten exakt rekonstruierten Bildes mit dem zumindest einen null-rekonstruierten Bild (108).

20. Bildgebungsverfahren nach Anspruch 12, wobei das Durchführen der apertur-gewichteten Rückprojektion der gefalteten Projektionsdaten unter Verwendung einer Apertur-Gewichtungsfunktion, die zumindest einige gefaltete redundante Projektionsdaten mit gefalteten Projektionsdaten, die innerhalb des exakten Rekonstruktionsfensters (38) liegen, gewichtet kombiniert, Folgendes umfasst:

Rekonstruieren der erfassten Projektionsdaten, gewichtet durch eine erste Aperturfunktion, die im Wesentlichen dem exakten Rekonstruktionsfenster (38) entspricht, zu einem nicht-redundanten exakt rekonstruierten Bild;
Vorwärtsprojizieren des nicht-redundanten exakt rekonstruierten Bildes, um einen simulierten Projektionsdatensatz zu erzeugen, der sich außerhalb des exakten Rekonstruktionsfensters (38) erstreckt; und
Kombinieren des simulierten Projektionsdatensatzes mit den erfassten Projektionsdaten, um einen Nullprojektionsdatensatz (108) zu bilden.

21. Bildgebungsverfahren nach Anspruch 20, wobei das Durchführen der apertur-gewichteten Rückprojektion der gefalteten Projektionsdaten unter Verwendung einer Apertur-Gewichtungsfunktion, die zumindest einige gefaltete redundante Projektionsdaten mit gefalteten Projektionsdaten, die innerhalb des exakten Rekonstruktionsfensters (38) liegen, gewichtet kombiniert, weiterhin Folgendes umfasst:

Rekonstruieren des Nullprojektionsdatensatzes (108), gewichtet durch eine erweiterte Aperturfunktion, die sich über einen breiteren Aperturbereich erstreckt als die erste Aperturfunktion, zu einem ersten null-rekonstruierten Bild;
Rekonstruieren des durch die erste Aperturfunktion gewichteten Nullprojektionsdatensatzes (108) zu einem zweiten null-rekonstruierten Bild; und
Kombinieren des nicht-redundanten exakt rekonstruierten Bildes, des ersten null-rekonstruierten Bildes und des zweiten null-rekonstruierten Bildes, um das rekonstruierte Bild mit Beiträgen von den redundanten Projektionsdaten zu bilden.

22. Bildgebungsverfahren nach Anspruch 21, wobei das Kombinieren des nichredundanten exakt rekonstruierten Bildes, des ersten null-rekonstruierten Bildes und des zweiten null-rekonstruierten Bildes Folgendes umfasst:

Subtraktives Kombinieren des zweiten null-rekonstruierten Bildes mit dem ersten null-rekonstruierten Bild, um eine Bildkorrektur zu erzeugen; und
Korrigieren des nicht-redundanten exakt rekonstruierten Bildes durch die Bildkorrektur.

## Revendications

1. Système d'imagerie de tomographie par ordinateur à faisceau conique, comprenant :

des moyens de balayage pour tomographie par ordinateur à faisceau conique (10) destinés à acquérir des données de projection à faisceau conique suréchantillonnées le long d'une trajectoire de source généralement hélicoïdale autour d'une région d'examen (14) ; et
des moyens de reconstruction exacte (40) comprenant :

des moyens de convolution (42) pour effectuer au moins une convolution des données de projection acquises, la convolution agissant sur des données de projection tombant à l'intérieur d'une fenêtre de reconstruction exacte (38) et sur au moins certaines données de projection redondantes tombant à l'extérieur de la fenêtre de reconstruction exacte (38) afin de produire des données de projection convolutées, et
des moyens de rétroprojection pondérés en ouverture (46, 66) pour effectuer une rétroprojection pondérée en ouverture des données de projection convolutées à l'aide d'une fonction de pondération d'ouverture qui combine par pondération au moins certaines données de projection redondantes convolutées avec des données de projection convolutées tombant à l'intérieur de la fenêtre de reconstruction exacte (38) pour générer une image reconstruite avec des contributions provenant de données de projection redondantes,

dans lequel les moyens de rétroprojection pondérés en ouverture (46, 66) utilisent une fonction de pondération d'ouverture variant de façon régulière dont la valeur est sélectionnée pour être substantiellement zéro à l'extérieur de la fenêtre de reconstruction exacte (38) et substantiellement un à l'intérieur de la fenêtre de reconstruction exacte (38) avec une région de transition de pondération d'ouverture régulière entre celles-ci.

2. Système d'imagerie selon la revendication 1, dans lequel les moyens de convolution (42) comprennent :

des moyens de réarrangement par hauteur (74) pour réarranger par hauteur des projections le long de plans K.

3. Système d'imagerie selon la revendication 2, dans lequel les moyens de réarrangement par hauteur (74) réarrangent des données de projection redondantes tombant à l'extérieur de la fenêtre de reconstruction exacte (38) pour des plans K qui sont pliés autour d'une ligne pi par rapport à des plans K correspondant à des données de projection complémentaires tombant à l'intérieur de la fenêtre de reconstruction exacte (38).

4. Système d'imagerie selon la revendication 2, dans lequel les moyens de réarrangement par hauteur (74) réarrangent des données de projection redondantes tombant à l'extérieur de la fenêtre de reconstruction exacte (38) pour des plans K qui sont complémentaires à des plans K correspondant à des données de projection tombant à l'intérieur de la fenêtre de reconstruction exacte (38).

5. Système d'imagerie selon la revendication 2, dans lequel la fenêtre de reconstruction exacte (38) est une fenêtre pi, et les moyens de rétroprojection pondérée en ouverture (46, 66) utilisent une fonction de pondération d'ouverture qui combine des données de projection redondantes situées entre un bord (90, 92) de la fenêtre pi et une ligne (94) connectant des points d'extrémité du bord de la fenêtre pi avec des données de projection complémentaires situées à l'intérieur de la fenêtre pi.

6. Système d'imagerie selon la revendication 1, dans lequel les moyens de rétroprojection pondérée en ouverture (46, 66) utilisent une fonction de pondération d'ouverture variant de façon régulière G(w) qui satisfait les critères :

$$G(w) = 0 \qquad \text{en} \qquad |w| = w_0,$$

$$G(w) = 0{,}5 \qquad \text{en} \qquad |w| = P/4,$$

et

$$G(w) = 1 \qquad \text{en} \qquad |w| \leq (0{,}5P - w_0),$$

où w est une coordonnée dans la direction d'angle conique, $w_0$ correspond à des bords d'ouverture d'un détecteur de rayonnement (16) des moyens de balayage de tomographie par ordinateur à faisceau conique (10), et P correspond à un pas d'hélice de la trajectoire généralement hélicoïdale.

7. Système d'imagerie selon la revendication 1, comprenant en outre :

des moyens (102) pour une projection directe d'une image reconstruite exacte formée en appliquant les moyens de reconstruction exacte (40) à un ensemble de données de projection exactes (100) tombant à l'intérieur de la fenêtre de reconstruction exacte (38) pour générer des données de projection simulées correspondant aux données de projection redondantes convolutées qui doivent être combinées par pondération avec des données de projection convolutées tombant à l'intérieur de la fenêtre de reconstruction exacte (38) ; et
des moyens (106) pour combiner les données de projection simulées et les données de projection convolutées pour générer un ensemble de données de projection nulles (108).

8. Système d'imagerie selon la revendication 7, dans lequel :

dans l'application des moyens de reconstruction exacte (40) à l'ensemble de données de projection exactes (100), les moyens de rétroprojection pondérés en ouverture (46, 66) appliquent une première fonction de pondération d'ouverture ($G_1$) à l'ensemble de données de projection exactes (100) pour produire une image reconstruite exacte non redondante, la première fonction de pondération d'ouverture ($G_1$) ayant une première bande passante d'ouverture ($\Delta w_{exact}$) correspondant substantiellement à la fenêtre de reconstruction exacte (38).

9. Système d'imagerie selon la revendication 8, dans lequel :

l'image reconstruite avec des contributions provenant de données de projection redondantes est générée par les moyens de rétroprojection pondérés en ouverture (46, 66) par :

la rétroprojection de l'ensemble de données de projection nulles (108) pondéré par la première fonction de pondération d'ouverture ($G_1$) pour générer une première image reconstruite nulle,
la rétroprojection de l'ensemble de données de projection nulles (108) pondéré par une fonction de pondération d'ouverture étendue ($G_2$) ayant une bande passante d'ouverture étendue englobant substantiellement à la fois la fenêtre de reconstruction exacte (38) et au moins un certain ensemble de données redondantes pour générer une deuxième image reconstruite nulle, et
la combinaison de l'image reconstruite exacte non redondante, de la première image reconstruite nulle et de la deuxième image reconstruite nulle.

10. Système d'imagerie selon la revendication 7, dans lequel les moyens de reconstruction exacte (40) reconstruisent l'ensemble de données de projection nulles (108) en au moins une image reconstruite nulle, l'image reconstruite avec des contributions provenant de données de projection redondantes étant générée en combinant une image reconstruite par les moyens de reconstruction (40) à partir des données de projection convolutées et ladite au moins une image reconstruite nulle.

11. Système d'imagerie selon la revendication 1, dans lequel les moyens de reconstruction exacte (40) effectuent une reconstruction qui satisfait les exigences de la transformée de Radon en trois dimensions pour des données de projection tombant à l'intérieur de la fenêtre de reconstruction exacte (38).

12. Procédé d'imagerie de tomographie par ordinateur à faisceau conique, comprenant les étapes suivantes :

acquérir des données de projection à faisceau conique suréchantillonnées le long d'une trajectoire de source généralement hélicoïdale autour d'une région d'examen (14) ; et
reconstruire des données de projection acquises tombant à l'intérieur d'une fenêtre de reconstruction exacte (38) et au moins certaines données de projection redondantes acquises tombant à l'extérieur de la fenêtre de reconstruction exacte (38) en une image reconstruite avec des contributions provenant de données de projection redondantes, la reconstruction comprenant les étapes suivantes :

appliquer une convolution aux données de projection acquises, la convolution agissant sur des données de projection acquises tombant à l'intérieur de la fenêtre de reconstruction exacte (38) et sur au moins certaines données de projection redondantes acquises tombant à l'extérieur de la fenêtre de reconstruction exacte (38) pour produire des données de projection convolutées, et
effectuer une rétroprojection pondérée en ouverture des données de projection convolutées à l'aide d'une fonction de pondération d'ouverture qui combine par pondération au moins certaines données de projection redondantes convolutées avec des données de projection convolutées tombant à l'intérieur de la fenêtre de reconstruction exacte (38) pour générer l'image reconstruite avec des contributions provenant de données de projection redondantes,

dans lequel la fonction de pondération d'ouverture comprend les étapes suivantes :

pondérer substantiellement par zéro des valeurs à l'extérieur de la fenêtre de reconstruction exacte (38) ;
pondérer substantiellement par un des valeurs à l'intérieur de la fenêtre de reconstruction exacte (38) ; et
pondérer des valeurs variant de façon régulière entre zéro et un dans une région de transition disposée à une périphérie de la fenêtre de reconstruction exacte (38).

13. Procédé d'imagerie selon la revendication 12, dans lequel la convolution comprend les étapes suivantes :

réarranger des données de projection pour des plans K ; et
effectuer au moins deux opérations de convolution, dont au moins l'une est effectuée après le réarrangement pour des plans K.

14. Procédé d'imagerie selon la revendication 13, dans lequel le réarrangement des données de projection le long des plans K comprend les étapes suivantes :

réarranger des données de projection tombant à l'intérieur de la fenêtre de reconstruction exacte (38) pour des premiers plans K ; et

réarranger des données de projection redondantes tombant à l'extérieur de la fenêtre de reconstruction exacte (38) pour des plans K modifiés qui sont complémentaires à au moins certains des premiers plans K.

**15.** Procédé d'imagerie selon la revendication 12, dans lequel la fenêtre de reconstruction exacte (38) est une fenêtre pi, et l'exécution d'une rétroprojection pondérée en ouverture comprend l'étape suivantes :

combiner par pondération des données de projection convolutées situées à l'extérieur de la fenêtre pi le long d'une périphérie des bords (90, 92) de la fenêtre pi avec des données de projection convolutées tombant à l'intérieur de la fenêtre de reconstruction exacte (38).

**16.** Procédé d'imagerie selon la revendication 12, dans lequel la fonction de pondération d'ouverture est une fonction de pondération d'ouverture unidimensionnelle.

**17.** Procédé d'imagerie selon la revendication 12, dans lequel l'exécution de la rétroprojection pondéré en ouverture comprend :

la pondération d'ouverture des données de projection réarrangées à l'aide d'une fonction de pondération d'ouverture lisse unidimensionnelle normalisée qui normalise des contributions de projections complémentaires, la fonction de pondération d'ouverture comportant :

des valeurs de pondération de normalisation variant de façon régulière pour des projections dans une région de transition substantiellement centrée sur les bords (90, 92) de la fenêtre de reconstruction exacte (38) ;
une valeur de pondération substantiellement égale à un à l'intérieur de la fenêtre de reconstruction exacte (38) et à l'extérieur de la région de transition ; et
une valeur de pondération substantiellement égale à zéro à l'extérieur de la fenêtre de reconstruction exacte (38) et à l'extérieur de la région de transition.

**18.** Procédé d'imagerie selon la revendication 12, comprenant en outre les étapes suivantes :

reconstruire une partie exacte de données de projection tombant à l'intérieur de la fenêtre de reconstruction exacte (38) en une image reconstruite exacte non redondante à l'aide d'une reconstruction qui satisfait les exigences de la transformée de Radon en trois dimensions ;
projeter directement l'image reconstruite exacte non redondante pour générer un ensemble de données de projection simulées s'étendant à l'extérieur de la fenêtre de reconstruction exacte (38) ; et
combiner l'ensemble de données de projection simulées et des données de projection convolutées comprenant au moins certaines données de projection redondantes convolutées pour former un ensemble de données de projection nulles (108).

**19.** Procédé d'imagerie selon la revendication 18, dans lequel l'exécution de la rétroprojection pondérée en ouverture des données de projection convolutées à l'aide d'une fonction de pondération d'ouverture qui combine par pondération au moins certaines données de projection redondantes convolutées avec des données de projection convolutées tombant à l'intérieur de la fenêtre de reconstruction exacte (38) comprend les étapes suivantes :

reconstruire l'ensemble de données de projection nulles (108) en au moins une image reconstruite nulle ; et
combiner l'image reconstruite exacte non redondante et ladite au moins une image reconstruite nulle (108).

**20.** Procédé d'imagerie selon la revendication 12, dans lequel l'exécution de la rétroprojection pondérée en ouverture des données de projection convolutées à l'aide d'une fonction de pondération d'ouverture qui combine par pondération au moins certaines données de projection redondantes convolutées avec des données de projection convolutées tombant à l'intérieur de la fenêtre de reconstruction exacte (38) comprend les étapes suivantes :

reconstruire les données de projection acquises pondérées par une première fonction d'ouverture correspondant substantiellement à la fenêtre de reconstruction exacte (38) en une image reconstruite exacte non redondante ;
projeter directement l'image reconstruite exacte non redondante pour générer un ensemble de données de projection simulées s'étendant à l'extérieur de la fenêtre de reconstruction exacte (38) ; et
combiner l'ensemble de données de projection simulées et les données de projection acquises pour former un ensemble de données de projection nulles (108).

**21.** Procédé d'imagerie selon la revendication 20, dans lequel l'exécution de la rétroprojection pondérée en ouverture des données de projection convolutées à l'aide d'une fonction de pondération d'ouverture qui combine par pondération au moins certaines données de projection redondantes convolutées avec des données de projection convolutées tombant à l'intérieur de la fenêtre de reconstruction exacte (38) comprend en outre les étapes suivantes :

reconstruire l'ensemble de données de projection nulles (108) pondéré par une fonction d'ouverture étendue qui couvre une plus grande plage d'ouverture que la première fonction d'ouverture en une première image reconstruite nulle ;
reconstruire l'ensemble de données de projection nulles (108) pondéré par la première fonction d'ouverture en une deuxième image reconstruite ; et
combiner l'image reconstruite exacte non redondante, la première image reconstruite nulle et la deuxième image reconstruite nulle pour former l'image reconstruite avec des contributions provenant de données de projection redondantes.

**22.** Procédé d'imagerie selon la revendication 21, dans lequel la combinaison de l'image reconstruite exacte non redondante, de la première image reconstruite nulle et de la deuxième image reconstruite nulle comprend les étapes suivantes :

combiner par soustraction la deuxième image reconstruite nulle à partir de la première image reconstruite nulle pour générer une correction d'image ; et
corriger l'image reconstruite exacte non redondante par la correction d'image.

FIG 1

FIG 2

EP 1 599 837 B1

EP 1 599 837 B1

42

```
┌─────────────────┐
│   1D fin_deriv   │
│    proc  70      │
└─────────────────┘
         │
         ▼
┌─────────────────┐
│  Con_angl_leng   │
│  Corr_proc  72   │
└─────────────────┘
         │
         ▼
┌─────────────────┐
│  Fwd_hght_rebin  │
│    Proc  74      │
└─────────────────┘
         │
         ▼
┌─────────────────┐
│   1D_Conv  76    │
└─────────────────┘
         │
         ▼
┌─────────────────┐
│  Rev_hght_rebin  │
│    Proc  80      │
└─────────────────┘
         │
         ▼
┌─────────────────┐
│    Inv_cos       │
│    Proc 82       │
└─────────────────┘
```

# FIG 3

FIG 4

FIG 5

FIG 6

FIG 7

EP 1 599 837 B1

Exact_proj
Dat_set 100

Exact Recon
Proc 40

Fwd Proj
Proc 102

Redundant
Dat_set 104

106

Σ

+

-

Null_proj
Dat_set 108

Synth_Redund
Dat_set 104'

FIG 8

FIG 9

EP 1 599 837 B1